# EUROPÄISCHE PATENTSCHRIFT

(11) **EP 4 154 861 B1**
(45) Veröffentlichungstag und Bekanntmachung des Hinweises auf die Patenterteilung: **07.05.2025**
(21) Anmeldenummer: 22196692.2
(22) Anmeldetag: 20.09.2022
(51) Int. Cl.: A61G 12/00, A61G 13/10

(54) **GERÄTESCHIENE ZUM HALTEN MEDIZINISCHER GERÄTE IN PATIENTENNÄHE, UND PATIENTENMÖBEL**
DEVICE RAIL FOR HOLDING MEDICAL DEVICES IN PATIENT PROXIMITY, AND PATIENT FURNITURE
RAIL D'APPAREIL POUR MAINTENIR DES DISPOSITIFS MÉDICAUX À PROXIMITÉ DU PATIENT ET MEUBLE POUR PATIENT

(30) Priorität: 28.09.2021 DE 202021105211 U
(43) Veröffentlichungstag der Anmeldung: 29.03.2023
(73) Patentinhaber: Ippen, Markus, 48155 Münster (DE)
(72) Erfinder: Ippen, Markus, 48155 Münster (DE)
(74) Vertreter: Patentanwälte Olbricht Buchhold Keulertz

(56) Entgegenhaltungen:
- DE-A1- 4 038 427
- DE-U1- 20 118 251
- FR-A1- 2 858 211
- US-A1- 2004 026 589
- US-A1- 2005 206 107
- US-A1- 2019 344 816
- US-A1- 2020 383 856

## Beschreibung

Die vorliegende Erfindung betrifft eine Geräteschiene zum Halten medizinischer Geräte in Patientennähe.

Aus der Praxis ist es bekannt, eines oder mehrere medizinische Geräte mittels einer Geräteschiene an einer Fahrtrage zu befestigen, es kann sich dabei z. B. um Geräte zur Sauerstoffversorgung, Infusionspumpen oder dergleichen handeln. Die Geräteschiene besteht erstens aus einer Profilleiste, die typischerweise geradlinig verläuft und einen rechteckigen Querschnitt aufweist, sowie zweitens aus mehreren Halterungen, die über die Länge der Profilleiste verteilt angeordnet sind und einerseits die Profilleiste festlegen sowie andererseits selbst an der Fahrtrage festgelegt sind.

Als Fahrtragen sind in diesem Zusammenhang Tragen bezeichnet, auf denen Patienten liegend transportiert werden können, wobei diese Tragen auf Rädern laufen. Fahrtragen werden im Rettungswesen benutzt, um Patienten zu einem Rettungswagen zu transportieren, oder später vom Rettungswagen ins Krankenhaus. Bei diesen Fahrtragen können die Fahrwerke z.B. faltbar sein, so dass der Patient auf der Trage in den Rettungswagen hinein transportiert werden kann und auch im Rettungswagen selbst auf diese Trage liegen bleiben kann, ohne umgebettet werden zu müssen.

Im Krankenhaus werden nicht Fahrtragen, sondern Patiententragen oder Patientenliegen benutzt, um Patienten zwischen ihrem Krankenzimmer / Patientenzimmer und beispielsweise einem Behandlungsraum oder Operationssaal liegend transportieren zu können. Es ist bekannt, auch an diesen Patiententragen oder Patientenliegen Geräteschienen vorzusehen, um medizinische Geräte in Patientennähe halten zu können. Die Patiententragen oder Patientenliegen können von Laien auch für ein Patientenbett gehalten werden, sie können beispielsweise ohne das oben erwähnte Falt-Fahrwerk ausgestaltet sein, sind aber üblicherweise schmaler als ein Patientenbett. Allerdings ist es aus der Praxis auch für Patientenbetten bekannt, die z. B. im Krankenhaus oder in einer Pflegeeinrichtung verwendet werden, medizinische Geräte mittels einer Geräteschiene in Patientennähe zu halten.

Abgesehen von den erwähnten Tragen, Liegen und Betten können medizinische Geräte mittels einer Geräteschiene auch an Patientenrollstühlen gehalten werden. Oder sie können mittels einer Geräteschiene an eigenen, fahrbaren Einheiten angeordnet sein, wie sie aus der Praxis beispielsweise unter der Bezeichnung "MobiDoc" bekannt sind und bei denen es sich um ein fahrbares Gestell handelt, das wahlweise an ein Bett, eine Trage oder dergleichen angekoppelt oder davon gelöst werden kann, wobei der "MobiDoc" im angekoppelten Zustand gemeinsam mit dem Bett, der Trage oder dergleichen verfahren werden kann.

Hilfsmittel, die einen Patienten im Liegen, Sitzen oder Stehen unterstützen, werden im Rahmen des vorliegenden Vorschlags als Patientenmöbel bezeichnet. Dies betrifft beispielsweise die oben genannten Tragen, Liegen, Betten und Rollstühle, aber auch Rollatoren und ähnliche Hilfsmittel. Die Patientenmöbel stellen dementsprechend stets die Tragkonstruktion dar, an welcher die Geräteschiene befestigt werden kann.

Alternativ zu den vorgenannten Patientenmöbeln, die stets eine mobile, nämlich fahrbare Installation der Geräteschiene darstellen, kann auch die Installation einer Geräteschiene an einer ortsfesten Tragkonstruktion vorgesehen sein, z. B. an einem ortsfest installierten Patientenmöbel wie einem fest stehenden, nicht fahrbaren Patientenbett, oder z. B. an einer Wand, die seitlich neben einem Patientenbett oder nahe dessen Kopfende verläuft.

Aus der Praxis sind Geräteschienen bekannt, die jeweils als Rechteck-Hohlprofil aus Edelstahl ausgestaltet sind. Mithilfe von Halterungen ist dieses Hohlprofil an einer Fahrtrage befestigt. Das Hohlprofil ist an den Halterungen durch Verschraubung befestigt, so dass das Hohlprofil dementsprechend Bohrungen aufweist, um die Schrauben aufnehmen zu können. Durch die Schraubbefestigung wird das Hohlprofil zwischen dem Schraubenkopf und der Halterung formschlüssig gegen Bewegungen in sämtlichen Richtungen gesichert. Die Bohrungen schwächen die Geräteschiene in mechanischer Hinsicht und erfordern einen zusätzlichen Bearbeitungsschritt bei der Herstellung. Wenn Bohrungen in der Vorder- bzw. Außenseite des Hohlprofils vorgesehen sind, also in der Seite, die von der Fahrtrage abgewandt ist, z.B. um eine Schraube durch das Hohlprofil durchstecken zu können, bieten die Bohrungen auch hygienische Nachteile und erschweren die Reinigung der Geräteschiene. Aus hygienischen Gründen sind die beiden Enden des Hohlprofils durch Abdecckappen aus Kunststoff geschlossen, wobei die Abdeckkappen auch als Verletzungsschutz dienen, da in der Praxis das Hohlprofil z. B. auf die gewünschte Länge gesägt sein kann und praktisch nachbearbeitungsfrei montiert sein kann, so dass es an seinen beiden Enden dementsprechend scharfkantig sein kann. Die Abdeckkappen werden stirnseitig in das Hohlprofil eingesteckt und halten durch Klemmung. Sie können im rauhen Alltagsbetrieb verloren gehen.

Aus der Praxis ist es alternativ bekannt, die Halterungen als Klemmbacken auszugestalten, so dass das Hohlprofil in den Halterungen fest eingespannt wird und dadurch gegen Bewegungen in fast allen Richtungen formschlüssig gesichert ist, allerdings nicht gegen Bewegungen in seiner Längsrichtung. Ausschließlich die Klemmkraft der Halterung sichert das Hohlprofil gegen seine unerwünschte Längsbewegung. Durch mechanische Einwirkungen wie Vibrationen, Fahrten über Pflastersteine oder einen ähnlichen, unebenen Untergrund oder dergleichen, die Klemmen sich lockern können. Weiterhin können hohe, auf das Hohlprofil einwirkende Kräfte dennoch eine Längsbewegung bewirken können, wenn nämlich die Klemmkraft überwunden wird, z.B. wenn die Fahrtrage gegen ein Hindernis anstößt oder der Rettungswagen mit der darin befindlichen Fahrtrage in einen Unfall verwickelt ist. Dies gilt insbesondere, da nicht nur das Eigengewicht des Hohlprofils zu berücksichtigen ist, sondern auch das Gewicht der am Hohlprofil befestigten Geräte, das aufgrund der Massenträgheit das Hohlprofil in Längsrichtung zu verschieben bestrebt ist. Auch bei dieser alternativen Halterung des Hohlprofils, die keine Bohrungen im Hohlprofil erfordert, müssen aus den genannten Gründen die beiden Enden des Hohlprofils verschlossen werden, typischerweise mit den erwähnten Kunststoff-Abdeckkappen.

Aus der US 2004 / 0 026 589 A1 ist ein Gestell bekannt, welches an einer Raumdecke aufgehängt ist und Strangpressprofile aufweist, die rechtwinklig aneinanderstoßen und mithilfe von Verbindungslaschen miteinander verbunden sind. Die Verbindungslaschen in Form dreidimensional verformter Bleche sind an die Querschnitte der jeweiligen Strangpressprofile angepasst. Je nach Konstruktion der vertikalen Streben des Gestells können medizinische Geräte wie Infusionspumpen direkt an diesen Streben gehalten werden, oder es können Konsolen, Schubläden oder dergleichen an den Streben befestigt sein, die ihrerseits zur Aufnahme medizinischer Geräte oder medizinischen Zubehörs dienen.

Aus der US 2005 / 0 206 107 A1 ist ein fahrbares Gestell bekannt, welches beispielsweise medizinische Infusionspumpen tragen kann und daher als Medizingeräteträgerwagen bezeichnet werden kann. Das Gestell weist einen horizontalen Arm auf, der an seinen beiden Enden Aufnahmeklauen in Form von C-förmig gebogen verlaufenden Formstücken trägt. Mittels der Aufnahmeklauen kann das Gestell an ein Patientenbett angekoppelt werden.

Aus der FR 2 858 211 A1 ist eine fahrbare Tragvorrichtung für medizinische Hilfsmittel der Patientenpflege bekannt. Ähnlich wie der Gegenstand der oben genannten US 2005 / 0 206 107 A1 kann auch dieses Gestell angekoppelt werden, jedoch nicht an ein Krankenhausbett, sondern an einen Wagen, der als Vorratslager für die medizinischen Hilfsmittel dient und als Lagerwagen bezeichnet wird. Das Gestell weist keine Geräteschiene auf, sondern Elemente in Form von Haken, Ablagetafeln, oder Stütztellern für Flaschen, um mittels dieser Elemente Hilfsmittel an dem Gestell zu halten. An einer horizontalen Traverse, die durch Schrauben oder Verschweißung fester Bestandteil des Gestells ist, sind Drahtkörbe zur Aufnahme von Abfallbeuteln gehalten.

Aus der DE 40 38 427 A1 ist ein Medizingeräteträgerwagen bekannt, der ebenfalls ähnlich wie der Gegenstand der oben genannten US 2005 / 0 206 107 A1 schnell mit Krankenbetten verschiedener Konstruktion verbunden und von ihnen wieder gelöst werden kann. Der Medizingeräteträgerwagen ist ein Gestell mit horizontalen und vertikalen Streben, wobei jeweils Formstücke dort zur Verbindung dienen, wo die einen an die anderen Streben anschließen. Der Medizingeräteträgerwagen weist keine Geräteschiene auf, an welche Medizingeräte angehängt werden können; vielmehr dient eine horizontale Geräteträgerplatte, die an den vertikalen Rohren höhenvariabel angebracht ist, dazu, die notwendigen medizinischen Geräte aufzunehmen.

Aus der US 2019 / 0 344 816 A1 ist ein medizinischer Organisationswagen bekannt. Dieser dient nicht dazu, medizinische Geräte in Patientennähe zu halten, sondern vielmehr dazu, medizinische Hilfsmittel wie Geräte oder Desinfektionsmittel zu transportieren und / oder in einem Vorratslager für den Gebrauch bereitzustellen. Der Organisationswagen weist einen geschlossenen Innenraum auf. Häufiger benötigte Hilfsmittel können außen an dem Wagen an einer Schiene gehalten werden, die somit als Geräteschiene bezeichnet werden kann. Diese Schiene wird auch als Klemmschiene bezeichnet, denn sie wird an zwei senkrechten Profilleisten des Organisationswagens durch Klemmung gehalten. Die Profilleisten weisen zu diesem Zweck Einbuchtungen auf, und die Geräteschiene ist an ihren beiden Enden mit Klauen versehen, die in diese Einbuchtungen eingreifen. Während eine Klaue unbeweglich an die Geräteschiene anschließt, ist die andere Klaue Teil eines Spannverschlusses, z.B. mit einer Exzenter-Mechanik, so dass die Geräteschiene werkzeuglos mittels des Spannverschlusses in beliebigen Höhen der Profilschienen an dem Organisationswagen festgelegt werden kann. Die Geräteschiene weist ein Hohlprofil auf und die beiden Klauen sind jeweils in das Hohlprofil eingesteckt; sie können mit Hilfe von Stahlstiften darin gesichert werden.

Der Erfindung liegt die Aufgabe zugrunde, eine gattungsgemäße Geräteschiene dahingehend zu verbessern, dass diese möglichst hygienisch ausgestaltet werden kann und eine sichere Festlegung der Profilleiste gewährleistet. Weiterhin liegt der Erfindung die Aufgabe zugrunde, ein Patientenmöbel anzugeben, das die Befestigung medizinischer Geräte auf möglichst sichere und hygienische Weise ermöglicht.

Diese Aufgabe wird durch eine Geräteschiene mit den Merkmalen des Anspruchs 1 gelöst sowie durch ein Patientenmöbel nach Anspruch 10. Vorteilhafte Ausgestaltungen sind in den Unteransprüchen beschrieben.

Die Erfindung schlägt mit anderen Worten vor, die Halterungen der Geräteschiene in der Art auszugestalten, dass sie die Profilleiste an deren beiden Enden jeweils ähnlich wie eine Kappe umgreifen, so dass diese beiden Halterungen als äußere Halterungen bezeichnet werden. Auf diese Weise ist die Profilleiste gegen Verlagerungen in sämtlichen Bewegungsrichtungen gesichert. Es ist dementsprechend nicht erforderlich, Bohrungen in die Profilleiste einzubringen, wie dies ansonsten für Verschraubungen o. ä. Befestigungsmöglichkeiten erforderlich wäre. Vorschlagsgemäß werden daher zwei Vorteile erreicht: durch die Festlegung der Profilleiste in Bezug auf sämtliche Bewegungsrichtungen wird die Profilleiste sicher gehalten. Dadurch, dass die beiden äußeren Halterungen die beiden Enden der Profilleiste haubenartig umgreifen, ist die Profilleiste an ihren beiden Enden abgedeckt, so dass auch nicht nachbearbeitete, ggf. scharfkantige Enden der Profilleiste keine Verletzungsgefahr darstellen können, sondern vielmehr verdeckt sind. Sollte die Profilleiste als Hohlprofil ausgestaltet sein, wird durch die beiden äußeren Halterungen das Hohlprofil an seinen beiden Enden abgedeckt, so dass hygienisch vorteilhaft keine Fremdkörper in das Hohlprofil eindringen können, wo sie für eine Reinigung schlecht zugänglich wären.

Die äußeren Halterungen können in einer Ausgestaltung eine im Querschnitt T-förmige Nut aufweisen. Der T-förmige Querschnitt der Nut bewirkt einen C-förmigen Querschnitt der Halterung. Dabei ist vorgesehen, dass die Nut in der so genannten Vorderseite der Halterung mündet, also in der Seite der Halterung, die von der Tagkonstruktion abgewandt ist. Auf diese Weise kann während der Montage der Geräteschiene unmittelbar überprüft werden, wie weit die Halterung auf die Profilleiste aufgeschoben bzw. die Profilleiste in die Halterung eingesteckt worden ist. Außerdem wird durch die an der Vorderseite mündende Nut ein Zugang von der Vorderseite geschaffen, so dass beispielsweise Schrauben o. ä. Befestigungselemente betätigt werden können, bevor die Profilleiste von der Halterung aufgenommen wird.

Die im Querschnitt T-förmige Nut erstreckt sich vorteilhaft nicht über die gesamte Länge der äußeren Halterung, so dass die Halterung einerseits ein offenes und andererseits ein geschlossenes Ende aufweist. In das offene bzw. Aufnahme-Ende kann die Profilleiste eingeführt werden, und das geschlossene Ende dient dazu, das Ende der Profilleiste abzudecken.

Je nach Länge der Geräteschiene und Gewicht der aufzunehmenden medizinischen Geräte sind Verformungen der Profilleiste im praktischen Betrieb nicht auszuschließen. Und daraus resultierende Beschädigungen der Profilleiste oder auch der medizinischen Geräte auszuschließen, kann in einer als vorteilhaft erachteten Ausgestaltung die Profilleiste durch eine oder mehrere zusätzliche Halterungen an ihrer jeweiligen Tragkonstruktion befestigt sein, wobei diese Halterungen als mittlere Halterungen bezeichnet sind, da sie sich zwischen den beiden äußeren Halterungen befinden. Auch diese mittleren Halterungen weisen eine im Querschnitt T-förmige Nut auf, so dass sie einen insgesamt etwa C-förmigen Querschnitt aufweisen, mit welchem sie die Profilleiste umgreifen. Die Nut durchsetzt in diesem Fall die mittleren Halterungen auf deren gesamter Länge.

Um die Befestigung der Halterungen an einer Tragkonstruktion zu erleichtern, können in der Rückseite der Halterungen Bohrungen vorgesehen sein. Die Bohrungen fluchtend mit der Mündung der T-förmigen Nut, die sich in der Vorderseite der Halterung befindet, so dass durch diese Mündung hindurch beispielsweise Befestigungselemente betätigt werden können.

Die erwähnten Bohrungen können vorteilhaft als Stufenbohrungen ausgestaltet sein, deren geringerer Durchmesser in der Rückseite der Halterung mündet und deren größerer Durchmesser in die Nut mündet, so dass beispielsweise eine Schraubenkopf in dem Abschnitt der Stufenbohrung angeordnet werden können, der den größeren Durchmesser aufweist, während sich der Schaft der Schraube durch den Abschnitt mit dem kleineren Durchmesser hindurch und bis in die Tragkonstruktion erstrecken kann.

Die Profilleiste kann vorteilhaft aus Edelstahl bestehen. Aufgrund seiner Korrosionsbeständigkeit ist in diesem Fall keine Oberflächenbehandlung in Form einer Lackierung oder dergleichen erforderlich, so dass medizinische Geräte wiederholt an der Geräteschiene, insbesondere an deren Profilleiste, befestigt und wieder gelöst oder entlang der Profilleiste verschoben werden können, ohne einen solchen Korrosionsschutz in Form einer Oberflächenbeschichtung zu beschädigen. Die Verwendung von Stahl lässt eine hohe Belastbarkeit einerseits durch das Gewicht der Geräte zu und andererseits auch durch deren Befestigungselemente, z.B. Klemmschrauben oder dergleichen, ohne unerwünschte Verformungen oder gar Beschädigungen der Profilleiste, so dass eine lange Nutzungsdauer der Geräteschiene ermöglicht wird. Die Ausgestaltung der Profilleiste als Hohlprofil verbessert die Biegesteifigkeit der Profilleiste und hilft insbesondere bei Patientenmöbeln, die zumindest kurzfristig angehoben und getragen werden müssen, das Gewicht möglichst gering zu halten.

Die Halterungen können in einer Ausgestaltung aus einer Aluminiumlage bestehen, so dass sie erstens auch ohne zusätzliche Maßnahmen gegen schädliche Korrosion geschützt sind, zweitens ein vergleichsweise geringes Gewicht aufweisen, und drittens beispielsweise im Vergleich zu Kunststoffen eine besonders hohe mechanische Belastbarkeit sicherstellen.

Ein besonders vorteilhaftes Patientenmöbel kann vorschlagsgemäß dadurch geschaffen werden, dass es mit einer Geräteschiene versehen wird, die nach dem vorliegenden Vorschlag ausgestaltet ist.

Dabei kann in einer als vorteilhaft erachteten Ausgestaltung vorgesehen sein, dass die Halterungen nicht unmittelbar an dem Patientenmöbel befestigt werden, sondern dass zumindest eine der Halterungen mithilfe eines Zwischenstücks an dem Patientenmöbel befestigt wird. Die Halterungen können daher wirtschaftlich vorteilhaft in größerer Stückzahl als Gleichteile hergestellt werden. Je nach Ausgestaltung des Patientenmöbels kann es möglich sein, eine Halterung unmittelbar an dem Patientenmöbel festzulegen, beispielsweise mit einem Rahmen oder dergleichen des Patientenmöbels zu verschrauben. In vielen Fällen wird es allerdings vorteilhaft sein, die Geräteschiene an eine Geometrie des Patientenmöbels anzupassen.

Diese Anpassung wird durch die erwähnten Zwischenstücke ermöglicht, die einerseits an die Geometrie des Patientenmöbels angepasst sind, und zwar individuell an die jeweilige Montagestelle, wo eine Halterung der Geräteschiene an dem Patientenmöbel befestigt werden soll. Auch kann ein Zwischenstück einen Ausleger aufweisen, der dem Patientenmöbel in Art einer Momentenstütze anliegt und Kräfte aufnimmt, die bestrebt sind, die Profilleiste der Geräteschiene um ihre Längsachse zu drehen, wenn eines oder mehrere medizinische Geräte an die Profilleiste angehängt sind. Andererseits bieten die Zwischenstücke eine Montagefläche, an der die Halterung der Geräteschiene befestigt werden kann. Aus hygienischen Gründen ist diese Montagefläche so ausgestaltet, dass ihr die Halterung der Geräteschiene möglichst spaltfrei angelegt werden kann. Beispielsweise können sowohl die Rückseite der Halterung als auch die Montagefläche des Zwischenstücks jeweils plan ausgestaltet sein.

Die Montage der Geräteschiene kann beispielsweise in den folgenden Schritten erfolgen: zunächst werden die Halterungen an den Zwischenstücken befestigt. Dann wird die Profilleiste in die Halterungen eingebracht, was einfach ist, weil die Zwischenstücke samt der damit verbundenen Halterungen frei beweglich sind. Anschließend werden die Zwischenstücke mitsamt der daran gehaltenen Geräteschiene an dem Patientenmöbel befestigt. Die Lage der Befestigungspunkte, die zur Befestigung der Zwischenstücke an dem Patientenmöbel dienen, sind dabei an den Zwischenstücken derart gewählt, dass sie auch nach der Montage der Geräteschiene problemlos zugänglich sind. Durch die beschriebene Vorgehensweise, bei welcher zunächst die Geräteschiene mit den Zwischenstücken verbunden wird, ist sichergestellt, dass die Halterungen perfekt ausgerichtet sind, um darin die Profilschiene aufzunehmen.

Eine alternative Möglichkeit, die Geräteschiene mithilfe von Zwischenstücken an einem Patientenmöbel zu befestigen, besteht darin, dass zunächst eine äußere und die mittlere Halterung mitsamt ihrer Zwischenstücke montiert werden, wobei zunächst die Halterungen fest an den Zwischenstücken befestigt werden, die Zwischenstücke dann jedoch noch nicht endgültig an der Trage festgelegt werden. Dann kann die Profilschiene eingeschoben werden, erforderliche Lagekorrekturen der beiden bereits montierten Zwischenstücke können dabei noch vorgenommen werden. Anschließend wird das dritte Zwischenstück montiert, in dem es zunächst mit der daran befindlichen Halterung auf das Ende der Profilschiene aufgeschoben wird und anschließend werden dieses sowie die beiden anderen Zwischenstücke endgültig an der Trage befestigt wird.

Vorteilhaft kann sichergestellt sein, dass die Profilleiste einer Geräteschiene möglichst spielfrei in den Halterungen angeordnet ist, so dass Klappergeräusche, aber auch unerwünschte, auf die medizinischen Geräte einwirkende Vibrationen oder Stöße vermieden werden, die ansonsten aus der Beweglichkeit der Profilschiene in den Halterungen resultieren könnten. Hierzu können verschiedene, dem Fachmann an sich bekannte Maßnahmen vorgesehen sein. Beispielsweise kann eine mit einer Feder belastete Kugel in einer Halterung gegen die Profilleiste drücken, oder eine gebogene Blattfeder kann in eine Halterung eingelegt sein, um die Profilleiste zu drücken, oder es kann ein Dämpfungselement - z. B. aus Kunststoff - in der Halterung zu demselben Zweck angeordnet sein. Eine Gewindebohrung in der Halterung kann in der Nut münden, in welcher die Profilleiste aufgenommen ist und dazu dienen, mittels einer Schraube, z. B. einer Madenschraube, die Profilleiste in der Halterung spielfrei zu klemmen.

Die Erfindung wird anhand der rein schematischen Darstellungen nachfolgend näher erläutert. Dabei zeigt
- Fig. 1: ein Ausführungsbeispiel einer Geräteschiene perspektivische Ansicht,
- Fig. 2 bis 4: unterschiedliche Ansichten einer äußeren Halterung,
- Fig. 5 bis 7: Ansichten ähnlich den Fig. 2 bis 4 einer mittleren Halterung, und
- Fig. 8: eine separate Einheit, die zur Schaffung eines Patientenmöbels wahlweise mit dem Patientenmöbel verbunden oder von diesem getrennt werden kann.

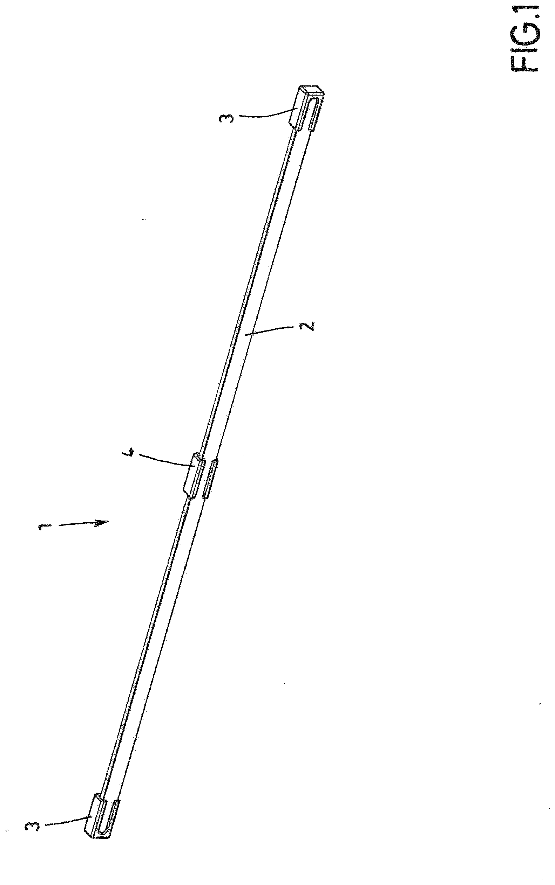

Fig. 1 zeigt eine Geräteschiene 1, die zur Verwendung im medizinischen Bereich bestimmt ist und an einem Patientenmöbel wie z. B. einer Fahrtrage befestigt werden kann. Die Geräteschiene 1 besteht einerseits aus einer Profilleiste 2, die als Rechteck-Hohlprofil aus Edelstahl ausgestaltet ist, und andererseits aus zwei unterschiedlichen Typen von Halterungen 3 und 4, die jeweils als Formteile aus Aluminium ausgestaltet sind. Zwei identisch ausgestaltete äußere Halterungen 3 umfassen die beiden Enden der Profilleiste 2 jeweils haubenartig, so dass durch das Zusammenwirken beider äußerer Halterungen 3 die Profilleiste 2 gegen Bewegungen in sämtlichen Richtungen formschlüssig gesichert ist. Die mittlere Halterung 4 muss daher nicht Längsverschiebungen der Profilleiste 2 verhindern, sie sichert die Profilleiste 2 vielmehr gegen Durchbiegungen in sämtlichen Biegerichtungen.

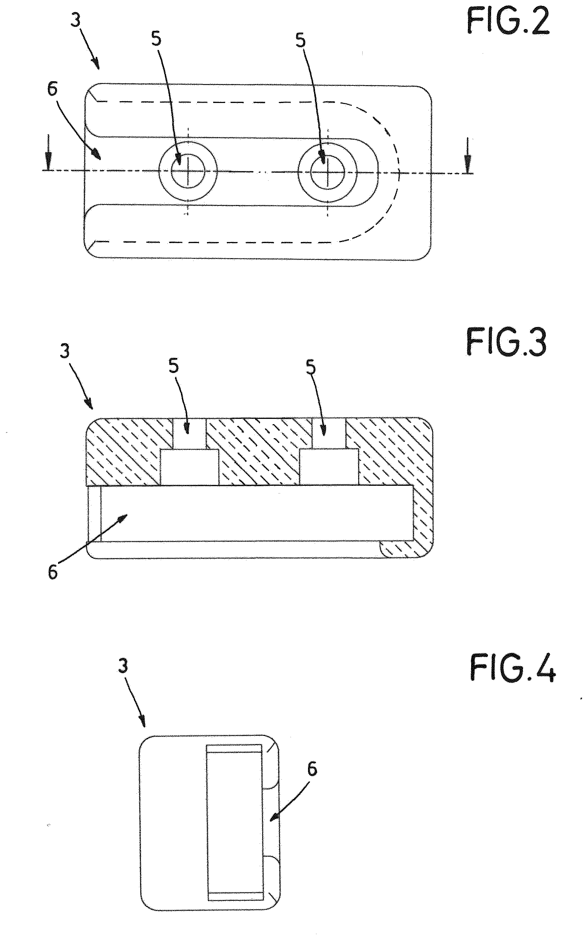

Die Fig. 2 bis 4 zeigen Ansichten einer äußeren Halterung 3. Zwei gewindelose Stufenbohrungen 5 dienen zur unmittelbaren Befestigung der Halterung 3 an einer Tragkonstruktion, oder an einem Zwischenstück zugunsten einer mittelbaren Befestigung an Tragkonstruktion wie zum Beispiel an einem Patientenmöbel. Abweichend von dem dargestellten Ausführungsbeispiel können auch weitere Bohrungen in der Halterung 3 vorgesehen sein, beispielsweise in Form von Gewindebohrungen, so dass mittels einer von der Rückseite her erfolgenden Verschraubung die Halterung 3 anstelle einer Mutter mit der Schraube zusammenwirken und auf diese Weise festgelegt werden kann. Oder es kann eine Gewindebohrung dazu dienen, die Profilleiste 2 zu beaufschlagen und spielfrei in der Halterung 3, 4 festzulegen. Dementsprechend kann durch die Anordnung von mehr Bohrungen als den beiden dargestellten Stufenbohrung und 5 die Halterung 3 für viele unterschiedliche Befestigungsarten vorbereitet sein, so dass die äußeren Halterungen 3 wirtschaftlich vorteilhaft in größerer Stückzahl als Gleichteile hergestellt werden können.

In Fig. 2 ist durch eine gestrichelte Kontur angedeutet, wie mittels eines T-Fräsers eine Nut 6 mit T-förmigem Querschnitt in den ursprünglich quaderförmigen Materialblock eingebracht worden ist. Am Ende der Nut 6 verläuft diese gestrichelte Kontur aufgrund der Drehbewegung des Fräsers halbkreisförmig. Um zu gewährleisten, dass das Ende der Profilleiste 2 von der Halterung 3 hygienisch vorteilhaft überdeckt wird, wird die Profilleiste 2 an ihren beiden Enden nicht geradlinig und quer abgeschnitten, sondern verjüngt sich vielmehr an ihren beiden Enden. Diese Verjüngung kann beispielsweise V-förmigem oder polygonal ausgestaltet sein, beispielsweise können die beiden Enden der Profilleiste 2 aber auch zu einem gerundeten Verlauf nachbearbeitet werden, mit demselben Radius, den der Fräser aufweist. Die gestrichelte Kontur entspricht daher in einem solchen Fall bis auf eine geringe maßliche Toleranz auch der Kontur, welche die Profilleiste 2 an ihren beiden Enden aufweist. Hierdurch wird ein möglichst dichter Anschluss der Profilleiste 2 an die äußere Halterung 3 ermöglicht, so dass hygienisch vorteilhaft das Eindringen von Verschmutzungen in das Hohlprofil der Profilleiste 2 vermieden wird.

Aufgrund ihres T-förmigen Querschnitts mündet die Nut 6 in der Vorderseite der äußeren Halterung 3, so dass die Stufenbohrungen 5 und die darin befindlichen Befestigungselemente, z.B. Schraubenköpfe, von der Vorderseite der Halterung 3 her problemlos zugänglich sind.

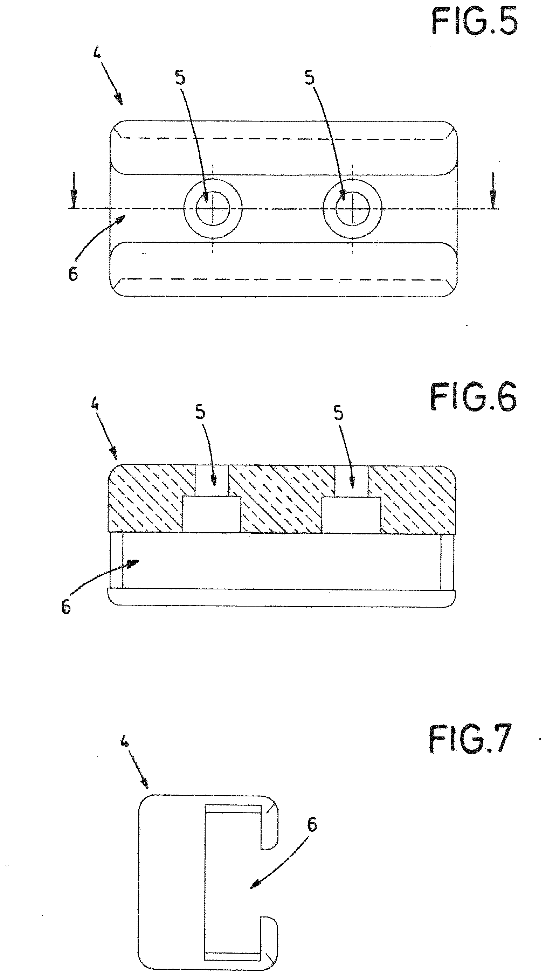

Die Fig. 5 bis 7 zeigen Ansichten ähnlich den Fig. 2 bis 4, jedoch von der mittleren Halterung 4. Der oben erwähnte T-Fräser ist in diesem Fall komplett durch den Materialblock hindurchgeführt worden, so dass die T-förmige Nut 6 die mittlere Halterung 4 auf voller Länge durchsetzt und nicht nur an einem, sondern an beiden Enden der mittleren Halterung 4 mündet. Die Außenabmessungen der beiden unterschiedlichen Typen von Halterungen 3 und 4 sind bei dem dargestellten Ausführungsbeispiel der Geräteschiene 1 gleich, so dass zugunsten einer wirtschaftlichen Fertigung stets gleiche Materialblöcke, z.B. aus einem Aluminiumwerkstoff, bereitgestellt werden können, deren Bearbeitung sich dann lediglich durch den unterschiedlichen Verfahrweg des Fräsers unterscheidet, um entweder eine äußere Halterung 3 oder eine mittlere Halterung 4 herzustellen.

Die aus Aluminium bestehenden Halterungen 3 und 4 sowie die aus Edelstahl bestehende Profilleiste 2 können so eng toleriert und so passgenau gefertigt werden, dass die Profilleiste 2 weitestgehend spielfrei und somit klapperfrei in den Halterungen 3, 4 gehalten ist. Insbesondere wenn Geräte an der Profilleiste 2 befestigt sind, wird diese innerhalb der Halterungen 3, 4 in dem durch das geringe Spiel möglichen Ausmaß verkantet werden und nicht mehr klappern.

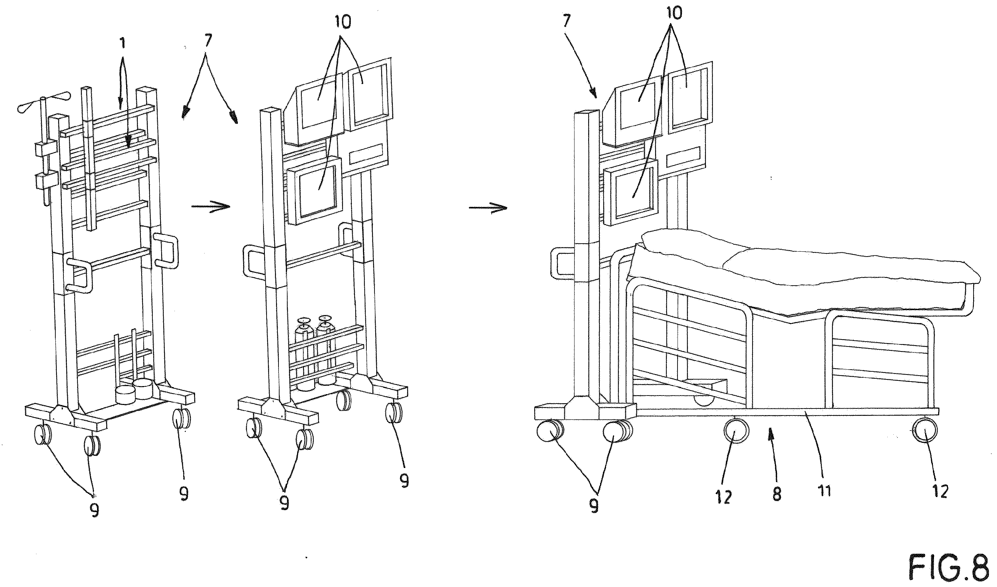

Fig. 8 zeigt in drei Schritten, wie eine separate, fahrbare Einheit 7 als Teil eines Patientenmöbels 8 eingesetzt werden kann. Links ist die fahrbare Einheit 7 separat dargestellt. Es handelt sich dabei um eine in der Praxis als "MobiDoc" bezeichnetes Gestell, welches im Wesentlichen aufrecht ausgerichtet ist und ein Fahrwerk mit eigenen Laufrädern 9 sowie mehrere Geräteschienen 1 aufweist.

**In** der Mitte von Fig. 8 ist dargestellt, dass diese fahrbare Einheit 7 mit mehreren medizinischen Geräten 10 bestückt worden ist, die an den Geräteschienen 1 befestigt worden sind daher in dieser Darstellung die Geräteschienen 1 verdecken.

Fig. 8 zeigt rechts, wie in einem Krankenhaus die fahrbare Einheit 7 an eine Patiententrage 11 angekoppelt worden ist, die eigene Laufräder 12 aufweist. Durch die Kopplung mit der fahrbaren Einheit 7 ist aus der Patiententrage 11 das Patientenmöbel 8 geschaffen worden, bei dem die medizinischen Geräte 10 in der Nähe des Patienten angeordnet sind.

### Bezugszeichen:

- 1: Geräteschiene
- 2: Profilleiste
- 3: Äußere Halterung
- 4: Mittlere Halterung
- 5: Stufenbohrung
- 6: Nut
- 7: Fahrbare Einheit
- 8: Patientenmöbel
- 9: Laufräder der Einheit 7
- 10: Medizinisches Gerät
- 11: Patiententrage
- 12: Laufräder der Patiententrage 11

## Patentansprüche

1. Geräteschiene (1), die dazu bestimmt ist, medizinische Geräte (10) in Patientennähe zu halten,
wobei die Geräteschiene (1) eine langgestreckte Profilleiste (2) aufweist,
sowie Halterungen (3, 4), die zur Festlegung an einer in der Nähe des Patienten und zur Befestigung der Geräteschiene vorgesehenen Tragkonstruktion bestimmt sind,
**dadurch gekennzeichnet,**
**dass** die Profilleiste (2) an ihren beiden Enden in zwei äußeren Halterungen (3) aufgenommen ist, welche das jeweilige Ende der Profilleiste (2) jeweils haubenartig übergreifen.

2. Geräteschiene nach Anspruch 1,
**dadurch gekennzeichnet,**
**dass** die äußeren Halterungen (3) eine im Querschnitt T-förmige Nut (6) aufweisen, die jeweils in einer von der Tragkonstruktion abgewandten Vorderseite der Halterung (3) münden,
derart, dass die äußere Halterung (3) im Bereich der Nut (6) einen C-förmigen Querschnitt aufweist.

3. Geräteschiene nach Anspruch 2,
**dadurch gekennzeichnet,**
**dass** die Nut (6) sich lediglich jeweils über einen Anteil der Länge der äußeren Halterungen (3) erstreckt, derart, dass die äußere Halterung (3) ein Aufnahme-Ende aufweist, in welches die Profilleiste einführbar ist, sowie ein gegenüberliegendes, geschlossenes Ende.

4. Geräteschiene nach einem der vorhergehenden Ansprüche,
**gekennzeichnet durch**
eine mittlere Halterung (4), die zwischen den beiden äußeren Halterungen (3) angeordnet ist,
und die eine im Querschnitt T-förmige Nut (6) aufweist, derart,
dass die mittlere Halterung (4) einen C-förmigen Querschnitt aufweist,
wobei die mittlere Halterung (4) die Profilleiste (2) umgreift und die T-förmige Nut (6) die mittlere Halterung (4) auf deren gesamter Länge durchsetzt.

5. Geräteschiene nach einem der Ansprüche 2 bis 4,
**dadurch gekennzeichnet,**
**dass** die Halterungen (3, 4) eine Rückseite aufweisen, die dazu bestimmt ist, der Tragkonstruktion zugewandt angeordnet zu werden,
sowie eine Vorderseite, die dazu bestimmt ist, von der Tragkonstruktion abgewandt angeordnet zu werden, wobei, mit die Mündung der Nut (6) fluchtend, Bohrungen die Rückseite der Halterungen (3, 4) durchsetzen.

6. Geräteschiene nach Anspruch 5,
**dadurch gekennzeichnet,**
**dass** die Bohrungen als Stufenbohrungen (5) ausgestaltet sind, deren größerer Durchmesser in der Nut (6) mündet und deren kleinerer Durchmesser in der Rückseite der Halterung (3, 4) mündet.

7. Geräteschiene nach einem der vorhergehenden Ansprüche,
**dadurch gekennzeichnet,**
**dass** die Profilleiste (6) als Rechteck-Hohlprofil aus Edelstahl ausgestaltet ist.

8. Geräteschiene nach Anspruch 7,
**dadurch gekennzeichnet,**
**dass** sich die Profilleiste (6) an ihren beiden Enden verjüngt.

9. Geräteschiene nach einem der vorhergehenden Ansprüche,
**dadurch gekennzeichnet,**
**dass** die Halterungen (3, 4) aus einer Aluminiumlegierung bestehen.

10. Patientenmöbel (8), das zur Unterstützung eines Patienten im Liegen, Sitzen oder Stehen bestimmt ist,
und an welchem eine Geräteschiene (1) angeordnet ist, die nach einem der vorhergehenden Ansprüche ausgestaltet ist.

11. Patientenmöbel nach Anspruch 10,
**dadurch gekennzeichnet,**
**dass** wenigstens eine der Halterungen (3, 4) der Geräteschiene (1) mithilfe eines Zwischenstücks an dem Patientenmöbel (8) befestigt ist.

## Claims

1. Device rail (1) which is intended to hold medical devices (10) in the proximity of a patient,
wherein the device rail (1) has an elongate profile strip (2),
and holders (3, 4) which are intended to be fixed to a supporting structure provided in the vicinity of the patient and for the fastening of the device rail,
**characterized**
**in that** the profile strip (2) is received at its two ends in two outer holders (3) which engage over the respective end of the profile strip (2) in each case in a cover-like manner.

2. Device rail according to Claim 1,
**characterized**
**in that** the outer holders (3) have a slot (6) which is T-shaped in cross section, which in each case open to a front side of the holder (3) facing away from the supporting structure,
in such a way that the outer holder (3) has a C-shaped cross section in the region of the slot (6).

3. Device rail according to Claim 2,
**characterized**
**in that** the slot (6) in each case extends only over a proportion of the length of the outer holders (3),
in such a way that the outer holder (3) has a receiving end, into which the profile strip is insertable, and an opposite, closed end.

4. Device rail according to one of the preceding claims,
**characterized by**
a middle holder (4) which is arranged between the two outer holders (3),
and which has a slot (6) which is T-shaped in cross section,
in such a way
that the middle holder (4) has a C-shaped cross section,
wherein the middle holder (4) engages around the profile strip (2) and the T-shaped slot (6) passes through the middle holder (4) over the entire length thereof.

5. Device rail according to one of Claims 2 to 4,
**characterized**
**in that** the holders (3, 4) have a rear side which is intended to be arranged facing the supporting structure,
and a front side which is intended to be arranged facing away from the supporting structure,
wherein bores pass through the rear side of the holders (3, 4), in alignment with the opening of the slot (6).

6. Device rail according to Claim 5,
**characterized**
**in that** the bores are designed as stepped bores (5), the larger diameter of which opens to the slot (6) and the smaller diameter of which opens to the rear side of the holder (3, 4).

7. Device rail according to one of the preceding claims,
**characterized**
**in that** the profile strip (6) is designed as a rectangular hollow profile composed of stainless steel.

8. Device rail according to Claim 7,
**characterized**
**in that** the profile strip (6) tapers at its two ends.

9. Device rail according to one of the preceding claims,
**characterized**
**in that** the holders (3, 4) consist of an aluminium alloy.

10. Item of furniture (8) for a patient which is intended to support a patient while lying, sitting or standing,
and on which a device rail (1) designed according to one of the preceding claims is arranged.

11. Item of furniture for a patient according to Claim 10,
**characterized**
**in that** at least one of the holders (3, 4) of the device rail (1) is fastened to the item of furniture (8) for a patient by means of an intermediate piece.

## Revendications

1. Rail pour appareils (1) destiné à supporter des appareils médicaux (10) à proximité du patient,
le rail pour appareils (1) présentant une baguette profilée allongée (2),
ainsi que des supports (3, 4) destinés à être fixés à une structure porteuse prévue à proximité du patient et pour la fixation du rail pour appareils,
**caractérisé en ce que**
la baguette profilée (2) est reçue à ses deux extrémités dans deux supports extérieurs (3) qui recouvrent respectivement l'extrémité respective de la baguette profilée (2) à la manière d'un capot.

2. Rail pour appareils selon la revendication 1,
**caractérisé en ce que**
les supports extérieurs (3) présentent une rainure (6) à section transversale en forme de T qui débouche respectivement dans un côté avant du support (3) détourné de la structure porteuse,
de telle sorte que le support extérieur (3) présente une section transversale en forme de C dans la zone de la rainure (6).

3. Rail pour appareils selon la revendication 2,
**caractérisé en ce que**
la rainure (6) s'étend uniquement sur une partie de la longueur de chacun des supports extérieurs (3),
de telle sorte que le support extérieur (3) présente une extrémité de réception dans laquelle la baguette profilée peut être insérée, ainsi qu'une extrémité opposée fermée.

4. Rail pour appareils selon l'une quelconque des revendications précédentes,
**caractérisé par**
un support central (4) agencé entre les deux supports extérieurs (3),
et qui présente une rainure (6) de section transversale en forme de T, de telle sorte que
le support central (4) présente une section transversale en forme de C,
le support central (4) entourant la baguette profilée (2) et la rainure en forme de T (6) traversant le support central (4) sur toute sa longueur.

5. Rail pour appareils selon l'une quelconque des revendications 2 à 4,
**caractérisé en ce que**
les supports (3, 4) présentent un côté arrière destiné à être agencé tourné vers la structure porteuse,
ainsi qu'un côté avant destiné à être agencé détourné de la structure porteuse, des alésages traversant le côté arrière des supports (3, 4) en étant alignés avec l'embouchure de la rainure (6).

6. Rail pour appareils selon la revendication 5,
**caractérisé en ce que**
les alésages sont conçus sous forme d'alésages étagés (5) dont le plus grand diamètre débouche dans la rainure (6) et dont le plus petit diamètre débouche dans le côté arrière du support (3, 4).

7. Rail pour appareils selon l'une quelconque des revendications précédentes,
**caractérisé en ce que**
la baguette profilée (6) est conçue sous forme de profilé creux rectangulaire en acier inoxydable.

8. Rail pour appareils selon la revendication 7,
**caractérisé en ce que**
la baguette profilée (6) se rétrécit à ses deux extrémités.

9. Rail pour appareils selon l'une quelconque des revendications précédentes,
**caractérisé en ce que**
les supports (3, 4) sont constitués d'un alliage d'aluminium.

10. Meuble pour patient (8) destiné à soutenir un patient en position couchée, assise ou debout,
et sur lequel est agencé un rail pour appareils (1) qui est conçu selon l'une quelconque des revendications précédentes.

11. Meuble pour patient selon la revendication 10,
**caractérisé en ce que**
au moins l'un des supports (3, 4) du rail pour appareils (1) est fixé au meuble pour patient (8) à l'aide d'une pièce intermédiaire.
